# EUROPEAN PATENT APPLICATION

(11) **EP 0 815 869 A1**
(43) Date of publication of application: **07.01.1998**
(21) Application number: 96906012.8
(22) Date of filing: 14.03.1996
(51) Int. Cl.: A61K 38/18, A61K 38/19, A61K 47/14, A61K 47/18, A61K 47/20, A61K 47/24, A61K 47/26, A61K 47/32, A61K 47/34, A61K 47/36, A61K 47/38

(54) **METHOD OF PREVENTING TPO ADSORPTION AND STABLE TPO-CONTAINING COMPOSITION**

(30) Priority: 15.03.1995 JP 56249/95
(71) Applicant: Kirin Brewery Company, Ltd., Tokyo 104 (JP)
(72) Inventor: OTSUKI, Naoki Kirin Brewery Company, Limited, Takasaki-shi Gunma-ken 370-12 (JP)
(74) Representative: UEXKÜLL & STOLBERG
(86) International application number: JP9600636
(87) International publication number: WO9628182

(57) **Abstract**

A TPO-containing composition comprising a TPO protein having no sugar-chain portion and at least one pharmaceutically acceptable additive selected from the group consisting of proteins, cellulose derivatives, sulfated polysaccharides, surfactants, polyvinyl alcohol, macrogols and aminoacetic acid; and a method of preventing TPO from being absorbed on a container wall by using the above composition containing such additives, thereby preventing the titer reduction caused by the adsorption.

SEQUENCE LISTING

INFORMATION FOR SEQ ID NO:1:
(i) SEQUENCE CHARACTERISTICS:
(A) LENGTH: 332 amino acids
(B) TYPE: amino acid
(ii) MOLECULE TYPE: protein
(vi) ORIGINAL SOURCE:
(A) ORGANISM: human (Homo sapiens)
(xi) SEQUENCE DESCRIPTION: SEQ ID NO:1:
Ser Pro Ala Pro Pro Ala Cys Asp Leu Arg Val Leu Ser Lys Leu Leu
1               5                   10                  15
Arg Asp Ser His Val Leu His Ser Arg Leu Ser Gln Cys Pro Glu Val
            20                  25                  30
His Pro Leu Pro Thr Pro Val Leu Leu Pro Ala Val Asp Phe Ser Leu
        35                  40                  45
Gly Glu Trp Lys Thr Gln Met Glu Glu Thr Lys Ala Gln Asp Ile Leu
    50                  55                  60
Gly Ala Val Thr Leu Leu Leu Glu Gly Val Met Ala Ala Arg Gly Gln
65                  70                  75                  80
Leu Gly Pro Thr Cys Leu Ser Ser Leu Leu Gly Gln Leu Ser Gly Gln
                85                  90                  95
Val Arg Leu Leu Leu Gly Ala Leu Gln Ser Leu Leu Gly Thr Gln Leu
            100                 105                 110
Pro Pro Gln Gly Arg Thr Thr Ala His Lys Asp Pro Asn Ala Ile Phe
        115                 120                 125
Leu Ser Phe Gln His Leu Leu Arg Gly Lys Val Arg Phe Leu Met Leu
    130                 135                 140
Val Gly Gly Ser Thr Leu Cys Val Arg Arg Ala Pro Pro Thr Thr Ala
145 150 55 160
Val Pro Ser Arg Thr Ser Leu Val Leu Thr Leu Asn Glu Leu Pro Asn
                165                 170                 175
Arg Thr Ser Gly Leu Leu Glu Thr Asn Phe Thr Ala Ser Ala Arg Thr
            180                 185                 190
Thr Gly Ser Gly Leu Leu Lys Trp Gln Gln Gly Phe Arg Ala Lys Ile
        195                 200                 205
Pro Gly Leu Leu Asn Gln Thr Ser Arg Ser Leu Asp Gln Ile Pro Gly
    210                 215                 220
Tyr Leu Asn Arg Ile His Glu Leu Leu Asn Gly Thr Arg Gly Leu Phe

225                 230                 235                 240
Pro Gly Pro Ser Arg Arg Thr Leu Gly Ala Pro Asp Ile Ser Ser Gly
                245                 250                 255
Thr Ser Asp Thr Gly Ser Leu Pro Pro Asn Leu Gln Pro Gly Tyr Ser
            260                 265                 270
Pro Ser Pro Thr His Pro Pro Thr Gly Gln Tyr Thr Leu Phe Pro Leu
        275                 280                 285
Pro Pro Thr Leu Pro Thr Pro Val Val Gln Leu His Pro Leu Leu Pro
    290                 295                 300
Asp Pro Ser Ala Pro Thr Pro Thr Pro Thr Ser Pro Leu Leu Asn Thr
305                 310                 315                 320
Ser Tyr Thr His Ser Gln Asn Leu Ser Gln Glu Gly
                325                 330

INFORMATION FOR SEQ ID NO:2:
  (i) SEQUENCE CHARACTERISTICS:
     (A) LENGTH: 535 base pairs
     (B) TYPE: nucleic acid
     (C) STRANDEDNESS: double
     (D) TOPOLOGY: linear
  (ii) MOLECULE TYPE: synthetic DNA
  (vi) ORIGINAL SOURCE:
     (A) ORGANISM: human (Homo sapiens)
  (xi) SEQUENCE DESCRIPTION: SEQ ID NO:2:
CTAGAAAAAA CCAAGGAGGT AATAAATA                                    28
ATG AAA AGT CCT GCA CCA CCT GCA TGT GAT TTA CGG GTC CTG TCT AAA    76
Met Lys Ser Pro Ala Pro Pro Ala Cys Asp Leu Arg Val Leu Ser Lys
        +1              5                   10
CTG CTG CGC GAC TCT CAC GTG CTG CAC TCT CGT CTG TCC CAG TGC CCG   124
Leu Leu Arg Asp Ser His Val Leu His Ser Arg Leu Ser Gln Cys Pro
 15                  20                  25                  30
GAA GTT CAC CCG CTG CCG ACC CCG GTT CTG CTT CCG GCT GTC GAC TTC   172
Glu Val His Pro Leu Pro Thr Pro Val Leu Leu Pro Ala Val Asp Phe
                 35                  40                  45
TCC CTG GGT GAA TGG AAA ACC CAG ATG GAA GAG ACC AAA GCT CAG GAC   220
Ser Leu Gly Glu Trp Lys Thr Gln Met Glu Glu Thr Lys Ala Gln Asp
             50                  55                  60
ATC CTG GGT GCA GTA ACT CTG CTT CTG GAA GGC GTT ATG GCT GCA CGT   268
Ile Leu Gly Ala Val Thr Leu Leu Leu Glu Gly Val Met Ala Ala Arg

65                  70                  75
GGC CAG CTT GGC CCG ACC TGC CTG TCT TCC CTG CTT GGC CAG CTG TCT     316
Gly Gln Leu Gly Pro Thr Cys Leu Ser Ser Leu Leu Gly Gln Leu Ser
    80                  85                  90
GGC CAG GTT CGT CTG CTG CTC GGC GCT CTG CAG TCT CTG CTT GGC ACC     364
Gly Gln Val Arg Leu Leu Leu Gly Ala Leu Gln Ser Leu Leu Gly Thr
95                  100                 105                 110
CAG CTG CCG CCA CAG GGC CGT ACC ACT GCT CAC AAG GAT CCG AAC GCT     412
Gln Leu Pro Pro Gln Gly Arg Thr Thr Ala His Lys Asp Pro Asn Ala
                115                 120                 125
ATC TTC CTG TCT TTC CAG CAC CTG CTG CGT GGC AAA GTT CGT TTC CTG     460
Ile Phe Leu Ser Phe Gln His Leu Leu Arg Gly Lys Val Arg Phe Leu
            130                 135                 140
ATG CTG GTT GGC GGT TCT ACC CTG TGC GTT CGT CGG GCG CCG CCA ACC     508
Met Leu Val Gly Gly Ser Thr Leu Cys Val Arg Arg Ala Pro Pro Thr
        145                 150                 155
ACT GCT GTT CCG TCT TAATGAAAGC TT                                   535
Thr Ala Val Pro Ser
    160

## Description

### BACKGROUND OF THE INVENTION

### 1. Field of the Invention

This invention relates to a stable composition which contains a TPO protein having no sugar chain moiety, more particularly, to a stable TPO-containing composition which is effective in preventing the loss or titer reduction of the TPO as active component caused by its adsorption on the wall of a container, or by its association, polymerization or the like. This invention also relates to a method for preventing the reduction of a TPO titer caused by adsorption of TPO on the wall of a container charged with the composition.

### 2. Disclosure of Related Art

Human TPO (thrombopoietin) is a protein cloned as a ligand of Mpl which is a member of the cytokine receptor superfamily (de Sauvage et al., Nature (London), vol.369, pp.533 - 565 (1994); Bartley, T.D. et al., Cell, vol.77, pp.1117 - 1124 (1994)). The Mpl ligands can be detected in sera and blood plasmas of animals (e.g., human, mouse, dog, etc.) suffering from thrombocytopenia, and its association with the formation of megakaryocytes and platelets has been already confirmed.

To develop a therapeutic agent for use in the treatment of thrombocytopenia, the present inventors have purified rat TPO from blood plasmas of thrombocytopenic rats by measuring an activity that stimulates the production of megakaryocytes from megakaryocyte progenitor cells highly purified from rat bone marrow, and have succeeded in cloning rat TPO cDNA and human TPO cDNA based on its partial amino acid sequence and in obtaining homogeneous human TPO in a large quantity by recombinant DNA techniques (H. Miyazaki et al., Exp. Hematol., vol.22, p.838 (1994)). The thus obtained human TPO has the same amino acid sequence as the above mentioned factor obtained as human Mpl ligand (see SEQ ID NO:1 described later).

The present inventors have found that the TPO of the present invention was effective for the treatment of thrombocytopenia, because thrombocytopenia inhibiting effect, thrombocytopoiesis enhancing effect and increased hematopoietic function were observed when said human TPO was administered to mice with thrombocytopenia in which bone marrow suppression has been induced by administration of a carcinostatic agent or an immunosuppressant, or by radiation or BMT.

Because of its high activity, TPO can be used in an extremely small amount, namely, it is normally administered several times a day in a dose of from 0.05 µg/kg body weight to 1 mg/kg body weight, preferably from 0.5 µg/kg body weight to 50 µg/kg body weight, as the active ingredient, depending on conditions, sex, and administration routes. Thus, it is necessary to produce TPO-containing pharmaceutical preparations using an extremely small quantity of TPO, but, in this case, it is unavoidable to cause reduction of a TPO titer due to the adsorption of TPO on containers (e.g., vials, ampoules, etc.) and the loss of the TPO ingredient caused by its adsorption onto infusion assemblies (bottles or tubes) when the preparations are mixed with a infusion liquid at the time of drip infusion.

In this context, the present inventors have studied a stable TPO-containing composition which can prevent adsorption of a TPO protein having no sugar chain moiety, with the aim of preventing reduction of a TPO titer when a pharmaceutical TPO preparation is produced or when it is mixed with a transfusion liquid. As a result, it has now been found that the addition of a pharmaceutically acceptable protein, cellulose derivative, sulfated polysaccharide, surface active agent, polyvinyl alcohol, macrogol (alias: polyethylene glycol), or aminoacetic acid (alias: glycine), to the TPO protein is effective for this purpose.

### SUMMARY OF THE INVENTION

Thus, according to the present invention, there is provided a method for the prevention of the reduction of a TPO titer caused by adsorption of TPO on the wall of a container charged with a composition that contains a TPO protein having no sugar chain moiety, which comprises adding to the composition at least one pharmaceutically acceptable additive selected from the group consisting of proteins, cellulose derivatives, sulfated polysaccharides, surface active agents (i.e., surfactants), polyvinyl alcohol, macrogols and aminoacetic acid.

The present invention also provides a TPO-containing composition which comprises a TPO protein having no sugar chain moiety and at least one pharmaceutically acceptable additive selected from the group consisting of proteins, cellulose derivatives, sulfated polysaccharides, surface active agents, polyvinyl alcohol, macrogols and aminoacetic acid.

### BRIEF DESCRIPTION OF THE DRAWINGS

Fig. 1 is a standard curve showing the relationship between a concentration of TPO(1-163)/*E. coli* and a corresponding absorbance value (450 nm/650 nm), as determined by ELISA.

Fig. 2 is a graph showing the change in % recovery of TPO(1-163)/*E. coli* over time when human serum albumin (HSA) is added in various concentrations.

### DETAILED DESCRIPTION OF THE INVENTION

As the TPO of the present invention, a protein having the amino acid sequence shown in SEQ ID NO:1 and having no sugar chain moiety may be used, and its production method is not particularly limited, provided that the product is an isolated protein having a high purity. Also useful as the TPO of the present invention is a protein which contains an amino acid sequence partially modified ( by substitution, deletion, insertion and/or addition) in the amino acid sequence shown in SEQ ID NO:1, but has no sugar chain moiety, provided that it maintains the TPO activity.

In other words, a protein substantially having the amino acid sequence shown in SEQ ID NO:1 and having no sugar chain moiety can also be used. The term "substantially having the amino acid sequence shown in SEQ ID NO:1" as used herein means that the "amino acid sequence resulting from partial substitution, deletion, insertion and/or addition in the amino acid sequence shown in SEQ ID NO:1, provided that it maintains the TPO activity" is included in addition to the amino acid sequence shown in SEQ ID NO:1.

It has been found that recombinant human TPO proteins keep a TPO activity even if amino acid residues of the C-terminal side of the amino acid sequence shown in SEQ TD NO:1 are deleted up to the position 152, or even if amino acid residues of the N-terminal side are deleted up to the position 6. Concrete data are shown in Table 1.

**Table 1**

| Derivative | Activity |
|---|---|
| Positions 1-231 | + |
| Positions 1-211 | + |
| Positions 1-191 | + |
| Positions 1-171 | + |
| Positions 1-163 | + |
| Positions 1-157 | + |
| Positions 1-156 | + |
| Positions 1-155 | + |
| Positions 1-154 | + |
| Positions 1-153 | + |
| Positions 1-151 | + |
| Positions 1-150 | - |
| Positions 7-163 | + |
| Positions 8-163 | - |
| Positions 13-231 | - |

Thus, the TPO proteins of the present invention are proteins having no sugar chain moiety, which contain an amino acid sequence corresponding to the 7 to 151 positions of the amino acid sequence shown in SEQ ID NO:1 and has the TPO activity. More specifically, the proteins include ones that have no sugar chain moiety and have a polypeptide chain of the positions 1-231, 1-211, 1-191, 1-171, 1-163, 1-157, 1-156, 1-155, 1-154, 1-153, 1-151 or 7-163 of the amino acid sequence shown in SEQ ID NO: 1.

Also included in the TPO proteins of the present invention are proteins having no sugar chain moiety and having a substitution, deletion, insertion and/or addition of at least one amino acid residue inside or outside the above mentioned 7-151 sequence shown in SEQ ID NO:1, to the extent that the TPO activity is not spoiled.

The TPO proteins having no sugar chain moiety of the present invention may include a protein in which at least the Ser¹ and Ala³ of the human TPO amino acid sequence shown in SEQ ID NO:1 are substituted by Ala and Val, respectively; a protein in which the Arg²⁵ is substituted by Asn; a protein in which the His³³ is substituted by Thr; a protein in which the Arg²⁵ is substituted by Asn and the Glu²³¹ is substituted by Lys; and proteins in which the polypeptide: Thr Ser Ile Gly Tyr Pro Tyr Asp Val Pro Asp Tyr Ala Gly Val His His His His His His is added to each C-terminus of the above described proteins.

Further included are proteins having no sugar chain moiety and having the deletion and/or addition of at least the following amino acid residues in the sequence shown in SEQ ID NO: 1, namely, a protein in which the His³³ is deleted; a protein in which the Gly¹¹⁶ is deleted; a protein in which the Arg¹¹⁷ is deleted; a protein in which a threonine residue is inserted between the His³³ and the Pro³⁴; a protein in which an alanine residue is inserted between the His³³ and the Pro³⁴; a protein in which a glycine residue is inserted between the His³³ and the Pro³⁴; a protein in which a glycine residue is inserted between the His³³ and the Pro³⁴ and the Pro³⁸ is substituted by Ser; a protein in which an asparagine residue is inserted between the Gly¹¹⁶ and the Arg¹¹⁷; a protein in which an alanine residue is inserted between the Gly¹¹⁶ and the Arg¹¹⁷; and a protein in which a glycine residue is inserted between the Gly¹¹⁶ and the Arg¹¹⁷. Still further examples of the TPO proteins of the present invention are a protein in which at least the Leu¹²⁹ is substituted by Arg; a protein in which the His¹³³ is substituted by Arg; a protein in which the Met¹⁴³ is substituted by Arg; a protein in which the Gly⁸² is substituted by Leu; a protein in which the Gly¹⁴⁶ is substituted by Leu; a protein in which the Ser¹⁴⁸ is substituted by Pro; a protein in which the Lys⁵⁹ is substituted by Arg; and a protein in which the Gln¹¹⁵ is substituted by Arg.

Also included as the TPO proteins having no sugar chain moiety of the present invention are non-glycosylated proteins in which methionine and lysine residues are respectively added to the positions -2 and -1 of the human TPO protein having the amino acid sequence shown in SEQ ID NO:1 and the above described derivatives; and proteins in which a methionine residue is attached at the position -1 of the human TPO protein having the amino acid sequence shown in SEQ ID NO:1 and the derivatives.

Preferably, the TPO proteins of the present invention may be obtained by isolating and purifying them from host cells transformed with a recombinant vector containing their cDNA, chromosomal DNA or chemically synthesized DNA. As the host, procaryotes (e.g., bacteria, preferably E. coli) can be used.

Proteins, cellulose derivatives, sulfated polysaccharides, surface active agents, polyvinyl alcohol, macrogols, aminoacetic acid, and the like can be exemplified as additives useful for the preparation of the stable TPO-containing composition of the present invention. Types of these additives are not particularly limited, provided that they can prevent adsorption of TPO on containers, tubes (e.g., infusion lines) or the like, made of glass, a resin or the like, which include materials set forth below.

As the proteins, human serum albumin, gelatin, bovine serum albumin, casein, collagen, human serum globulin and the like can be used.

As the cellulose derivatives, methylcellulose, hydroxypropylcellulose, hydroxyethylcellulose and the like can be used.

Examples of useful surface active agents include polyoxyethylene hydrogenated castor oil; polyoxyethylene castor oil; polyoxyethylene sorbitan fatty acid esters such as polysorbate 80, polyoxyethylene sorbitan monolaurate (alias: polysorbate 20) and the like; polyoxyethylene polyoxypropylene glycol; sorbitan fatty acid esters such as sorbitan monooleate and the like; sucrose fatty acid esters such as sucrose monolaurate and the like; egg yolk phospholipids such as lecithin from egg yolk and the like; aromatic quaternary ammonium salts such as benzethonium chloride, benzalkonium chloride and the like; alkyl sulfates such as sodium lauryl sulfate and the like.

As the sulfated polysaccharides, chondroitin sulfate sodium salt, heparin sodium, etc. are usable.

The above described additives as used in the present invention may be used in a concentration ranging from 0.001% to 10% when the TPO-containing composition is prepared in the form of an aqueous solution. Also, it is desirable to use these additives within the range of from 0.02 parts by weight to 10,000 parts by weight per part by weight of TPO protein as an active ingredient.

In addition, the TPO-containing composition of the present invention may also contain a diluent, a solubilizing agent, an antiseptic agent, an antioxidant, an excepient, an isotonicity or the like, depending on preparation purposes of the composition.

The stable TPO-containing composition of the present invention can be prepared in dosage forms such as solutions, suspensions, tablets, pills, capsules, granules or freeze-dried preparations, depending on various administration routes that include parenteral administration (e.g., injection), transpulmonary administration, transnasal administration and oral administration. The TPO and the additive as used in the present invention may be formulated such that they coexist in the same composition from the beginning, or alternatively the TPO and the additive may be separately preformulated and blended when used.

The following test examples and working examples will be provided to further illustrate the present invention.

### EXAMPLES

In the following test examples, the enzyme immunoassay of TPO was carried out as follows.

### Enzyme-linked immunosorbent assay (ELISA) using anti-human TPO monoclonal antibody

An anti-human TPO monoclonal antibody (i.e., subclone L3-1-54 of L3-1) as a solid phase antibody which recognizes the human TPO HT-1 region (corresponding to the 8 - 28 amino acid sequence shown in SEQ ID NO:1) was prepared in a concentration of 20 µg/ml in 50 mM carbonate buffer (pH 9.2) and dispensed in 50 µl portions into wells of a 96-well microtiter plate (Nunc-Immuno Plate MaxiSorp™, manufactured by InterMed, Ca.No. 4-42404). The solid phase antibody was thoroughly spread on the bottom of each well while shaking the plate on a microplate mixer (ADVANTEC TS-96, manufactured by Advantech Toyo, Japan). The plate was sealed with a plate seal (manufactured by SUMILON, Ca.No. MS-30020) and left at 37°C for 2 hours or at 4°C overnight to adsorb the solid phase antibody on the plate. Next, after washing with a washing buffer (20 mM Tris-HCl/0.5 M NaCl/0.1% Tween 20 (pH 7.5)), 300 µl of 4 X Block Ace (manufactured by Dainippon Pharmaceuticals, Japan, Ca No. UK-B25) was added to each well, and the plate was sealed with a plate seal and left at 37°C for 1 hour or at 4°C overnight. After washing 4 times with the washing buffer, 50 µl of a sample to be tested or a known concentration of TPO as a standard which was diluted with 10 X Block Ace, was added to each well, and the plate was shaken using a microplate mixer, sealed with a plate seal and then left at 37°C for 2 hours or at 4°C overnight. After completion of the reaction, the plate was washed 4 times with the washing buffer. Next, another anti-human TPO monoclonal antibody (i.e., subclone L4-1-31 of L4-1) as a primary antibody which recognizes the human TPO HT-2 region (corresponding to the 47-62 amino acid sequence shown in SEQ ID NO:1) was labeled with biotin, diluted to 500 ng/ml with 10 X Block Ace and dispensed in 50 µl portions into the wells, after which the plate was shaken on a microplate mixer, sealed with a plate seal and then left at 37°C for 2 hours or at 4°C overnight. After the completion of the reaction and the subsequent washing (x4) with the washing buffer, a peroxidase- labeled avidin (UltraAvidin™-Horseradish Peroxidase, manufactured by Leinco Technologies, Ca.No. A106) was diluted 2,000 folds in 10 X Block Ace and dispensed in 50 µl portions into the wells. The plate was shaken on a microplate mixer, sealed with a plate seal and then subjected to 1 hour of reaction at 37°C. After the completion of the reaction and the subsequent washing (x4) with the washing buffer, 100 µl of a color forming agent prepared by adding 1/100 volumes of a substrate solution to a TMBZ color former of the color developing kit for peroxidase (manufactured by SUMILON, Ca No. ML-1120T) was added to each well, and the plate was shaken on a microplate mixer, sealed with a plate seal and then subjected to the reaction at the room temperature. After about 30 minutes, 100 µl of a reaction termination solution was added to each well, and the place was shaken using a microplate mixer to stop the coloring reaction. Absorbances at wave lengths of 450 nm/650 nm were measured using an ELISA plate reader (THERMOmax, manufactured by Molecular Devices).

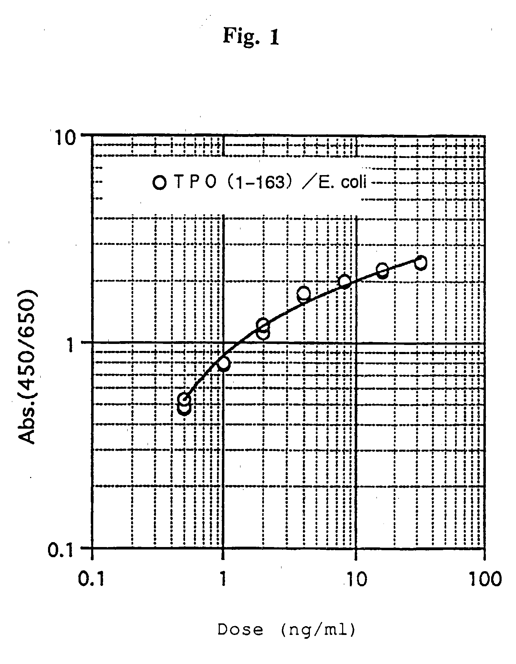

A standard curve was made based on absorbance values of known concentrations of TPO(1-163)/E. coli obtained by the procedure described in Reference Example (see Fig. 1).

In this connection, the subclone (L4-1-31) of the hybridoma L4-1 and the subclone (L3-1-54) of the hybridoma L3-1 used herein, which the subclones can both produce anti-human TPO monoclonal antibodies, have been deposited on December 27, 1994 with the National Institute of Bioscience and Human Technology, Agency of Industrial Science and Technology, Ministry of International Trade and Industry, Japan, under Accession Nos. FERM BP-4956 and FERM BP-4955, respectively. The above subclones have also been deposited with the Chinese depositary authority (CCTCC) under Accession No. CCTCC-C95003 for Mouse-Mouse hybridoma L4-31 and Accession No. CCTCC-C95002 for Mouse-Mouse hybridoma L3-1-54, respectively.

### Test Example 1

Test samples were prepared by adding human serum albumin to 10 mM Tris buffer (pH 7.5) to a final concentration of 0.005%, 0.01%, 0.02%, 0.05%, 0.1%, 0.2% or 0.5%. As a control sample, 10 mM Tris buffer (pH 7.5) alone was used.

1.5 µl of a solution containing 3 µg of TPO obtained by the procedure described later in Reference Example was put in a glass test tube which has been charged with 1,000 µl of each sample, and was then left at the room temperature. The solution was taken from the tube in 10 µl portions after 0.5 minutes, 1 hour or 2 hours, and the amount of TPO in the solution was measured by ELISA to calculate its recovery (%) based on the expected value.

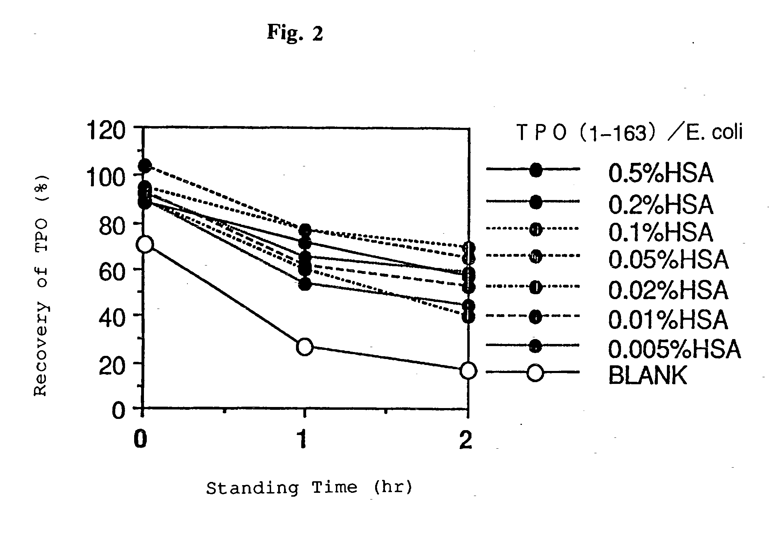

The results are shown in Fig. 2. In the drawing, BLANK indicates a case of 10 mM Tris buffer (pH 7.5) only and HSA indicates human serum albumin. It was found that human serum albumin had the adsorption preventing effect.

### Test Example 2

2.7 µl of a solution containing 3 µg of TPO [i.e., TPO(1-163)/E. coli] obtained by the procedure described in Reference Example was added to a glass test tube which has been charged with 1,000 µl of a solution prepared by dissolving each of the various additives shown in Table 2 in 10 mM Tris buffer (pH 7.5) to a final concentration of 0.1%, and the recovery after 24 hours of standing was measured by ELISA. The results are shown in Table 2.

**Table 2**

| Additives (amount added, 1 mg/ml) | Recovery (%) |
|---|---|
| human serum albumin | 41.3 |
| purified gelatin (type A) | 41.2 |
| purified gelatin (type B) | 24.9 |
| aminoacetic acid | 21.7 |
| methylcellulose | 44.0 |
| hydroxypropylcellulose | 51.5 |
| hydroxyethylcellulose | 23.3 |
| chondroitin sulfate sodium salt | 61.6 |
| heparin sodium | 41.4 |
| Macrogol 400 | 24.4 |
| polyvinyl alcohol (partially saponified) | 36.7 |
| polyoxyethylene hydrogenated castor oil 60 | 50.3 |
| polysorbate 80 | 67.8 |
| polyoxyethylene sorbitan monolaurate | 57.6 |
| polyoxyethylene(160) polyoxypropylene(30) glycol | 33.7 |
| sorbitan monooleate | 89.6 |
| sucrose monolaurate | 59.2 |
| egg yolk phospholipid | 20.8 |
| benzethonium chloride | 97.6 |
| sodium lauryl sulfate | 72.6 |
| no addition | 16.2 |

As shown in the table, all the additives tested could prevent the adsorption of TPO when contacted to glass test tubes. Production examples of the TPO-containing compositions of the present invention will be set forth below.

### Example 1

An aqueous solution that contains 1,000 µg of TPO, 0.025 g of human serum albumin and 87.66 mg of sodium chloride in 10 ml of 5 mM phosphate buffer (pH 6.0) was aseptically prepared and dispensed in 1 ml portions into vials which were subsequently sealed.

### Example 2

A pharmaceutical preparation was prepared by repeating the procedure of Example 1, except that 0.1 g of purified gelatin (type B) was used instead of 0.025 g of human serum albumin.

### Example 3

An aqueous solution that contains 2,500 µg of TPO, 10 mg of polysorbate 80 and 0.5 g of sorbitol in 10 ml of 1 mM citrate buffer (pH 6.0) was aseptically prepared and dispensed in 1 ml portions into vials which were subsequently sealed.

### Example 4

An aqueous solution that contains 2,500 µg of TPO, 10 mg of polyoxyethylene hydrogenated castor oil 60 and 81.82 mg of sodium chloride in 10 ml of 10 mM Tris buffer (pH 6.5) was aseptically prepared and dispensed in 1 ml portions into vials which were subsequently sealed.

As a reference example, a process for the production of TPO as an active ingredient of the present invention is described below.

### Reference Example: Example of the production of TPO(1-163)/E. coli in Escherichia coli

### (1) Construction of E. coli expression plasmid pAMG11-hMKT(1-163) for hMKT(1-163) and its expression in E. coli:

To express a protein having an amino acid sequence of the positions 1 through 163 shown in SEQ ID NO:1 (referred to as "TPO(1-163)/E. coli" hereinafter) in *E. coli*, a DNA fragment coding for the amino acid sequence was chemically synthesized using preferential codons for *E. coli*. In addition, a nucleotide sequence which encodes methionine and lysine residues newly added at the N-terminal side was ligated with the DNA fragment, and a DNA sequence encoding a stop codon was added to a site corresponding to the C-terminal side. SEQ ID NO:2 shows an amino acid sequence of the protein encoded by this DNA, namely the protein in which the Met-Lys are attached to the N-terminus of the 1-163 amino acid sequence shown in SEQ ID NO:1 (referred to as "hMKT(1-163)" hereinafter).

The hMKT(1-163) gene fragment synthesized as above has XbaI and HindIII restriction sites at its 5'-end and 3'-end, respectively, and it contains a ribosome binding site, an ATG initiation codon, a sequence encoding the amino acid sequence of hMKT(1-163), and a stop codon.

The above fragment was cloned into the XbaI-HindIII sites of the lactose-inducible expression vector, pAMG11. The pAMG11 vector is a low copy-number plasmid having a pR100-derived replication origin. The expression vector pAMG11 can be obtained from a plasmid pCFM1656 (ATCC No.69576, deposited on February 24, 1994) by causing a series of site-directed base mutations via mutagenesis accompanied with PCR. This plasmid has a BglII site (plasmid bp # 180) starting with immediately at the 5'-side of a plasmid replication promoter, PcopB, followed by a plasmid replication gene. The mutation of base pairs is shown in Table 3.

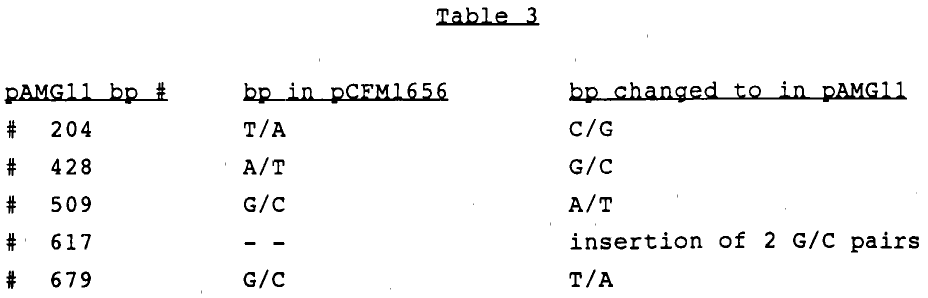

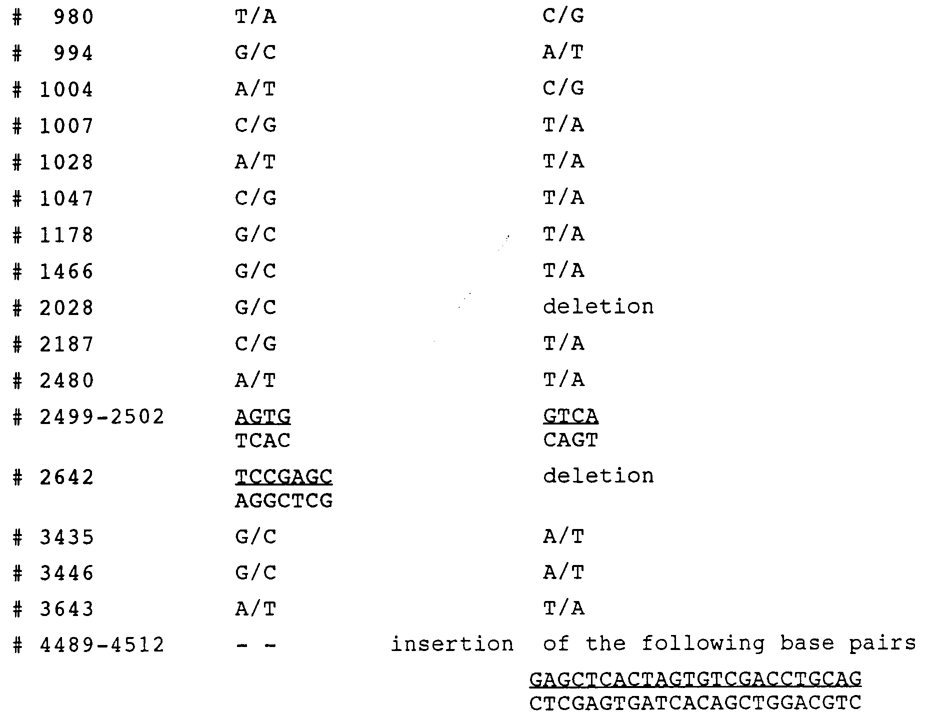

Next, the DNA sequence between the unique AatII and ClaI sites was replaced by the following oligonucleotide.

AatII (#4358)
5'      CTCATAATTTTTAAAAAATTCATTTGACAAATGCTAAAATTCTT-
3' TGCAGAGTATTAAAAATTTTTTAAGTAAACTGTTTACGATTTTAAGAA-

-GATTAATATTCTCAATTGTGAGCGCTCACAATTTAT   3'
-CTAATTATAAGAGTTAACACTCGCGAGTGTTAAATAGC 5'
ClaI (#4438)

Expression of the hMKT(1-163) gene introduced into pAMG11 can be induced by a synthetic lactose-inducible promoter such as a Ps4 promoter having the following sequence:

5' GACGTCTCATAATTTTTAAAAAATTCATTTGACAAATGCTAAA-
-ATTCTTGATTAATATTCTCAATTGTGAGCGCTCACAATTTATCGAT 3'

The Ps4 promoter-induced expression of hMKT(1-163) gene is repressed by the lactose repressor (Lac I) which is a product of the *E. coli* lac I gene.

Next, an *E. coli* strain K-12 containing laq I^{q} allele was transformed with the plasmid pAMG11-hMKT(1-163). The laq I^{q} allele has a mutation within the lac I promoter which increases expression of the Lac I gene, thereby resulting in more stringent control of protein expression by the Ps4 promoter. In consequence, in the absence of lactose, expression of hMKT(1-163) is repressed by Lac I. When lactose is added, the binding of the Lac I protein to the operator site of the Ps4 promoter decreases, and the transcription of the hMKT(1-163) gene is initiated by the Ps4 promoter. The *E. coli* used as the host cell in this example has been deposited with the ATCC on November 30, 1994 under ATCC No. 69717.

The *E. coli* strain (ATCC No. 69717) was transformed with the plasmid pAMG11-hMKT(1-163) and cultured under the following culture conditions.

### (2) Culture of a recombinant E. coli strain capable of expressing hMKT(1-163) and production of TPO(1-163)/E. coli:

The obtained transformant was cultured on LB medium at 30°C for approximately 12 hours. The cells were then aseptically transferred to a fermenter containing a batch medium (20 g/L yeast extract; 3.4 g/L citric acid; 15 g/L K₂HPO₄; 15 ml Dow P2000; 5 g/L glucose; 1 g/L MgSO₄-7H₂O; 5.5 ml/L trace metals; 5.5 ml/L vitamins). The cultivation was continued until an optical density (O.D.) of the culture reached 5.0 ± 1.0 at 600 nm. Then, a first feed medium (700 g/L glucose; 6.75 g/L MgSO₄-7H₂O) was fed while adjusting a feed rate at intervals of 2 hours in accordance with an established schedule. The addition of a second feed medium (129 g/L trypticase peptone; 258 g/L yeast extract) was started when the O.D. of the culture reached 20-25 at 600 nm. The addition of the second feed medium was maintained at a constant flow rate while the addition of the first feed medium was continued to be adjusted.

The temperature was maintained at approximately 30°C during the entire cultivation. The culture was kept at about pH 7 with addition of an acid or a base if necessary. The desired dissolved oxygen level was maintained by adjusting an agitation rate, an aeration rate and an oxygen influx rate in the fermenter. When the O.D. of the culture reached 57-63 at 600 nm, the addition of a third feed medium (300 g/L lactose) was introduced into the fermenter at a constant flow rate. The addition of the first feed medium was stopped and the flow rate of the second feed medium was changed to a new constant rate. The cultivation was continued over about ten hours after initiation of the addition of the third feed medium. At the end of the cultivation, the culture was cooled to 15 ± 5°C and the cells were harvested by centrifugation. The resulting pellet was stored at a temperature of -60°C or lower.

Purification of hMKT(1-163) thus produced in *E. coli* and production of TPO(1-163)/E. coli were carried out as follows.

1800 g of the cell pellet was suspended in about 18 liters of 10 mM EDTA and passed through a high pressure homogenizer at 15,000 psi. The broken cell suspension was centrifuged and the precipitate was resuspended in 10 L of 10 mM EDTA. The suspension was centrifuged and 200 g of the precipitate was solubilized in 2 L of 10 mM Tris buffer, pH 8.7, containing 8 M guanidine hydrochloride, 10 mM DTT and 5 mM EDTA. This solution was slowly diluted in 200 L of 10 mM CAPS, pH 10.5, containing 3 M urea, 30% glycerol, 3 mM cystamine and 1 mM cysteine.

The diluted solution was stirred slowly for 16 hr at the room temperature and the pH was adjusted to 6.8. After the adjustment of pH, the solution was clarified and loaded to a 2-L CM Sepharose column equilibrated with 10 mM sodium phosphate buffer, pH 6.8, containing 1.5 M urea and 15% glycerol. After loading, the column was washed with 10 mM sodium phosphate containing 15% glycerol, pH 7.2. hMKT(1-163) was eluted with a linear gradient from 0 M to 0.5 M sodium chloride in 10 mM sodium phosphate buffer, pH 7.2.

The fractions eluted from the CM Sepharose column were concentrated using a membrane (10,000 molecular weight cut off) and simultaneously buffer-exchanged with 10 mM sodium phosphate buffer, pH 6.5. The concentrated solution (protein: about 2 mg/ml) was treated with cathepsin C (protein substrate : enzyme = 500 : 1 (molar ratio)) for 90 minutes at the ambient temperature.

The reaction mixture was then loaded to a 1.2-L SP High Performance Sepharose column equilibrated with 10 mM sodium phosphate buffer, pH 7.2, containing 15% glycerol. After loading, a TPO active protein TPO(1-163)/E. coli in which the N-terminal Met-Lys was cleaved from the hMKT(1-163) was eluted with a linear gradient from 0.1 M to 0.25 M sodium chloride in 10 mM sodium phosphate, pH 7.2.

Ammonium sulfate was added to the eluate from the SP High Performance column to a concentration of 0.6 M. The eluate was then loaded to a 1.6-L Phenyl Toyopearl column (Toso Corp., Japan) equilibrated with 10 mM sodium phosphate buffer, pH 7.2, containing 0.6 M ammonium sulfate. A peak of the TPO(1-163)/E. coli was eluted with a linear gradient from 0.6 M to 0 M ammonium sulfate in 10 mM sodium phosphate, pH 7.2.

The resulting eluate from the Phenyl Toyopearl column was concentrated using a membrane (10,000 molecular weight cut off) and simultaneously buffer-exchanged with 10 mM Tris buffer, pH 7.5, containing 5% sorbitol.

## Claims

1. A thrombopoietin (TPO)-containing composition comprising a TPO protein having no sugar chain moiety and at least one pharmaceutically acceptable additive selected from the group consisting of proteins, cellulose derivatives, sulfated polysaccharides, surface active agents, polyvinyl alcohol, macrogols and aminoacetic acid.

2. The TPO-containing composition according to claim 1 wherein said additive is contained in an amount of from 0.001% to 10% when the composition is in the form of an aqueous solution.

3. The TPO-containing composition according to claim 1 or claim 2 wherein said protein is human serum albumin and/or gelatin.

4. The TPO-containing composition according to claim 1 or claim 2 wherein said cellulose derivative is at least one compound selected from the group consisting of methylcellulose, hydroxypropylcellulose and hydroxyethylcellulose.

5. The TPO-containing composition according to claim 1 or claim 2 wherein said surface active agent is at least one compound selected from the group consisting of polyoxyethylene hydrogenated castor oil, polyoxyethylene castor oil, a polyoxyethylene sorbitan fatty acid ester, a sorbitan fatty acid ester, a sucrose fatty acid ester, an egg yolk phospholipid, an aromatic quaternary ammonium salt, and an alkyl sulfate.

6. The TPO-containing composition according to claim 1 or claim 2 wherein said sulfated polysaccharide is chondroitin sulfate sodium salt and/or heparin sodium.

7. A method for preventing the reduction of a TPO titer caused by adsorption of TPO on the wall of a container charged with a composition that contains a TPO protein having no sugar chain moiety, which comprises adding to the composition at least one pharmaceutically acceptable additive selected from the group consisting of proteins, cellulose derivatives, sulfated polysaccharides, surface active agents, polyvinyl alcohol, macrogols and aminoacetic acid.
